# EUROPEAN PATENT SPECIFICATION

(11) **EP 2 606 814 B1**
(45) Date of publication and mention of the grant of the patent: **19.10.2016**
(21) Application number: 11828656.6
(22) Date of filing: 23.08.2011
(51) Int. Cl.: G06F 19/00, A61B 5/00, A61B 1/04, A61B 8/00, G06Q 50/00

(54) **MEDICAL SYSTEM, MEDICAL SYSTEM COMMUNICATIONS METHOD, MEDICAL IMAGE PHOTOGRAPHY DEVICE, AND SERVER**
MEDIZINISCHES SYSTEM, KOMMUNIKATIONSVERFAHREN FÜR DAS MEDIZINISCHE SYSTEM, VORRICHTUNG ZUR AUFNAHME MEDIZINISCHER BILDER UND SERVER DAFÜR
SYSTÈME MÉDICAL, PROCÉDÉ DE COMMUNICATION ENTRE SYSTÈMES MÉDICAUX, DISPOSITIF DE PHOTOGRAPHIE D'IMAGES MÉDICALES ET SERVEUR

(30) Priority: 29.09.2010 JP 2010219046
(43) Date of publication of application: 26.06.2013
(73) Proprietor: Olympus Corporation, Tokyo 192-8507 (JP)
(72) Inventor: OZAKI, Takashi, Tokyo 151-0072 (JP)
(74) Representative: Gunzelmann, Rainer
(86) International application number: PCT/JP2011/068900
(87) International publication number: WO 2012/043095

(56) References cited:
- EP-A1- 1 677 127
- EP-A2- 1 203 296
- WO-A2-2004/027676
- WO-A2-2007/143401
- JP-A- 2004 344 390
- JP-A- 2005 122 237
- JP-A- 2010 131 034
- US-A1- 2004 015 079
- US-A1- 2007 120 550
- US-A1- 2007 120 550
- OMA MWG-IM SWG: "Beverly hills, USA ; OMA-IM-2004-0027R01-Session-Reestablishmen t-ppt-quick-setup", OMA-IM-2004-0027R01-SESSION-REESTABLISHMEN T-PPT-QUICK-SETUP, OPEN MOBILE ALLIANCE (OMA), 4330 LA JOLLA VILLAGE DR., SUITE 110 SAN DIEGO, CA 92122 ; USA , 31 January 2004 (2004-01-31), pages 1-11, XP064060765, Retrieved from the Internet: URL:ftp/Public_documents/COM/IM/2004/ [retrieved on 2004-01-31]
- JEEHOON PARK ET AL: "Session Reestablishment ; OMA-IM-2004-0026R01-Session-Reestablishmen t-Quick-setup", OMA-IM-2004-0026R01-SESSION-REESTABLISHMEN T-QUICK-SETUP, OPEN MOBILE ALLIANCE (OMA), 4330 LA JOLLA VILLAGE DR., SUITE 110 SAN DIEGO, CA 92122 ; USA , 30 January 2004 (2004-01-30), pages 1-2, XP064060764, Retrieved from the Internet: URL:ftp/Public_documents/COM/IM/2004/ [retrieved on 2004-01-31]

## Description

### [Technical Field]

The present invention relates to a medical system and a communication method for a medical system, a medical image capturing apparatus, and a server.

### [Background Art]

As disclosed in Japanese laid-open Patent Publication No. 2010-131034 or the like for example, the DICOM (Digital Imaging and Communications in Medicine) standard has recently received a lot of attention both within and outside of hospitals. The DICOM is a standard for medical imaging and communication, which enables the exchange of image examination information of patients and the transmission of image data by interconnecting different types (multi-modalities) of digital imaging devices (modalities) by different manufacturers (multi-vendors) via the information network or the like.

In the DICOM standard, meaningful interconnection among the above-mentioned imaging devices is achieved according to the purpose of medical treatment, and it is expected that problems of the conventional imaging diagnostic system where films are mainly used (lack of storage space, film loss, late delivery, etc.) will be overcome and that new added values in the imaging diagnosis (digital imaging process, computer-assisted diagnosis, comprehensive imaging diagnosis, etc.) will be achieved.

However, in the above DICOM standard, examination interruption or examination resumption during a single examination on a modality side is not assumed. Thus, every time such an interruption occurs, it is treated as if another examination has started. For this reason, there have been technical problems in which the management of the medical images that are sent by a modality side and stored by a medical server is inconvenient.

For example, when an endoscope apparatus is used as a modality, the power of the endoscope apparatus is cut every time a scope is replaced with another one during a single examination which is being performed for the same patient. Accordingly, a series of single examinations is split before and after the power of the endoscope apparatus is cut, and the medical server recognizes the series of single examinations as separate examinations. For this reason, the management of the medical images or the like has been complicated and inconvenient as a user has had to manage medical images while constantly paying attention to the existence of examination interruptions.

Japanese laid-open Patent Publication No. 2010-131034 merely discloses a technique in which medical image data and order information are associated and stored in a portable medium in a DICOM-compatible medical image system such that the medical image data stored in the portable medium can be easily used when the portable medium is brought to another medical institution, but does not at all indicate a technical problem caused by interruption or resumption of an examination on the modality side.

Document US 2007/120550 A1 discloses an endoscope control system adapted to identify whether the previous system termination was normal or abnormal (e.g. accidental power shut-off), and in the event the previous system termination was abnormal, to quickly restart system devices necessary for observing an endoscopic image separately from and without having to wait for a longer restart of the entire endoscope control system. In the event the previous system termination was normal, the system devices are restarted in a normal sequence as part of the longer restart of the entire endoscope control system.

An object of the present invention is to provide a technique in which examination information such as medical images acquired in a single examination may be accurately recognized and precisely managed without being influenced by interruption and resumption of an examination or the like on a medical image generation apparatus side.

### [Means for Solving the Problems]

The above object is achieved by a medical system according to claim 1 and by a communication control method according to claim 4. Advantageous embodiments are defined in the dependent claims.

According to a first aspect a medical system is provided including a medical image capturing apparatus and a server that records a medical image transmitted from the medical image capturing apparatus, and the medical system includes: first examination status storage unit and second examination status storage unit for storing an examination status of under examination or examination complete in the medical image capturing apparatus in each of the medical image capturing apparatus and the server; first control unit provided with a function of notifying the server of the examination status in the medical image capturing apparatus and a function of shifting the medical image capturing apparatus to a status of examination complete according to an examination complete executive instruction from the server; and second control unit for generating the examination complete executive instruction that changes from the server the examination status of the medical image capturing apparatus according to information of the examination status received from the medical image capturing apparatus and a status of the server.

According to a second aspect a communication method for a medical system is provided including a medical image capturing apparatus and a server that records a medical image transmitted from the medical image capturing apparatus, and the method includes: a first step of notifying an examination status of under examination or examination complete in the medical image capturing apparatus from the medical image capturing apparatus to the server, and sharing information of the examination status between the medical image capturing apparatus and the server; a second step of notifying the examination status from the medical image capturing
apparatus to the server at communication reestablishment after communication interruption between the medical image capturing apparatus and the server; and a third step of changing, by the server, the examination status of the medical image capturing apparatus according to whether there is a change between the information of the examination status received in the server in the second step and the examination status of the server side.

According to a third aspect a medical image capturing apparatus that configures a medical system is provided, where the medical image capturing apparatus is connected to a server that stores a medical image acquired by examination, and the medical image capturing apparatus includes: communication unit for transmitting and receiving information with the server; examination status storage unit for storing an examination status of under examination or examination complete in the medical image capturing apparatus; and control unit for matching the examination status to an examination status of the server, the control unit being provided with an examination status notification function of notifying the server of the examination status and an examination complete execution instruction recognition function of terminating an examination of the medical image capturing apparatus according to an examination complete executive instruction from the server.

According to a fourth aspect a server that stores a medical image transmitted from a medical image capturing apparatus and configures a medical system is provided, where the server is connected to the medical image capturing apparatus, and the server includes: communication unit for transmitting and receiving information with the medical image capturing apparatus; examination status storage unit for storing an examination status of the server synchronized with an examination status of under examination or examination complete in the medical image capturing apparatus; and control unit provided with a function of
receiving a notice of the examination status from the medical image capturing apparatus to update the examination status storage unit and a function of sending to the medical image capturing apparatus an examination complete executive instruction to shift the medical image capturing apparatus to the examination complete when the server is forced to shift to the examination complete, and matching the examination status to an examination status of the medical image capturing apparatus.

According to a fifth aspect a method for controlling a medical image capturing apparatus that configures a medical system is provided, where the medical image capturing apparatus is connected to a server that stores a medical image acquired by examination, and the method includes: a step of detecting communication reestablishment after communication with the server is cut; a step of notifying to the server an examination status of under examination or examination complete in the medical image capturing apparatus when the communication reestablishment is detected; and a step of terminating an examination of the medical image capturing apparatus according to an examination complete executive instruction from the server.

According to a sixth aspect a method for controlling a server that stores a medical image transmitted from a medical image capturing apparatus and that configures a medical system is provided, where the server is connected to the medical image capturing apparatus, and the method includes: a step of transmitting and receiving information with the medical image capturing apparatus; a step of storing an examination status of the server synchronized with an examination status of under examination or examination complete in the medical image capturing apparatus; a step of receiving a notice of the examination status from the medical image capturing apparatus to update storage of the examination status in regard to the medical image capturing apparatus; and a step of sending to the medical image capturing apparatus an examination complete executive instruction to shift the medical image capturing apparatus to the examination complete when the server is forced to shift to the examination complete, and matching the examination status to an examination status of the medical image capturing apparatus.

### [Effects of the Invention]

According to the present invention, it becomes possible to provide a technique in which examination information such as medical images acquired in a single examination may be accurately recognized and precisely managed without being influenced by interruption and resumption of an examination or the like on a medical image generation apparatus side.

### [Brief Description of the drawings]

FIG. 1 is a block diagram of the overall composition of a medical system provided with a medical image capturing apparatus according to one embodiment of the present invention.
FIG. 2 is an explanatory schematic diagram of some types of scopes for a endoscope that are removable from a medical image capturing apparatus and a light source device of FIG. 1.
FIG. 3 is a schematic diagram depicting an example of the configuration of a modality-side status table provided for an endoscope apparatus of a medical system according to one embodiment of the present invention.
FIG. 4 is a schematic diagram depicting an example of the configuration of a server-side status table provided for an image server of a medical system according to one embodiment of the present invention.
FIG. 5 is a sequence diagram depicting a basic communication sequence in a medical system according to one embodiment of the present invention.
FIG. 6 is a sequence diagram depicting a basic communication sequence in a medical system according to one embodiment of the present invention.
FIG. 7 is a flowchart illustrating an example of the processing in which the states of an endoscope apparatus and an image server are coordinated in a medical system according to one embodiment of the present invention.
FIG. 8 is a sequence diagram illustrating an example of the processing in which the states of an endoscope apparatus and an image server are coordinated in a medical system according to one embodiment of the present invention.
FIG. 9 is a sequence diagram illustrating an example of the processing in which the states of an endoscope apparatus and an image server are coordinated in a medical system according to one embodiment of the present invention.
FIG. 10 is a sequence diagram illustrating an example of the processing in which the states of an endoscope apparatus and an image server are coordinated in a medical system according to one embodiment of the present invention.
FIG. 11 is a sequence diagram illustrating an example of the processing in which the states of an endoscope apparatus and an image server are coordinated in a medical system according to one embodiment of the present invention.
FIG. 12 is a flowchart of an example of the operation of an endoscope apparatus in a medical system according to another embodiment of the present invention.
FIG. 13 is a flowchart illustrating an example of the modification of a medical system according to another embodiment of the present invention.
FIG. 14 is a sequence diagram illustrating inconsistencies in the controlled state of images caused due to the communication interruption between an endoscope apparatus and an image server according to another additional embodiment of the present invention.
FIG. 15 is a flowchart illustrating an example of the processes performed when the communication is reestablished in an endoscope apparatus of a medical system according to another additional embodiment of the present invention.
FIG. 16 is a sequence diagram illustrating a reference example that relates to an embodiment of the present invention.
FIG. 17 is a sequence diagram illustrating a reference example that is related to an embodiment of the present invention.

### [Description of the Preferred Embodiment]

In one mode of the present embodiment, examination status is compaired and adjusted (i.e., a cross-reference of status) after communication is established and then is cut between a medical image capturing apparatus (processor) that generates a medical image and an image server that collects and manages medical images.

Accordingly, even if, for example, the communication between a processor and an image server is cut when the power source of the processor is cut in order to replace a scope during an examination in a medical image capturing apparatus such as an endoscope apparatus, it becomes possible to handle examination data such as medical images as a succession of a single examination before and after the communication is cut.

Some embodiments of the present invention will be described below in detail with reference to the drawings.

### (Embodiment 1)

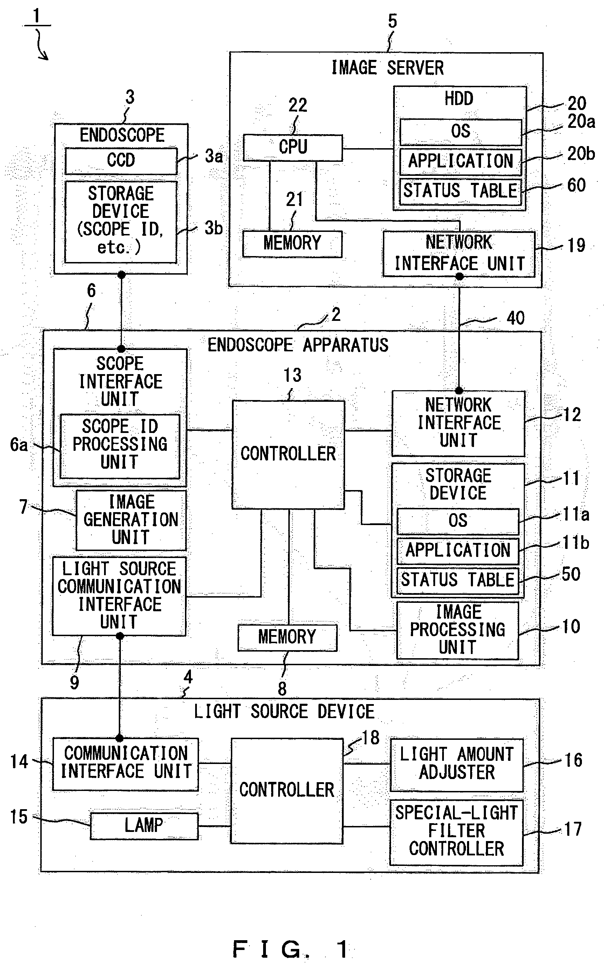

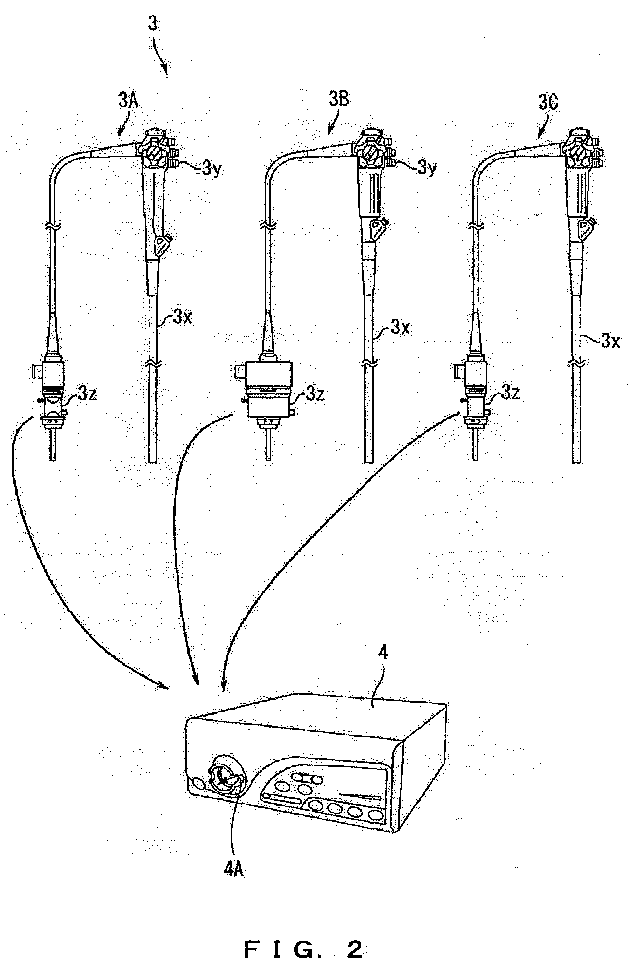

FIG. 1 is a block diagram of the overall composition of a medical system provided with a medical image capturing apparatus. FIG. 2 is an explanatory schematic diagram of some types of endoscopes that are removable from a medical image capturing apparatus and a light source device of FIG. 1.

As illustrated in FIG. 1, a medical system 1 according to the present embodiment is provided with a DICOM-supported endoscope apparatus 2 (medical image capturing apparatus) that configures a medical device, an endoscope that configures examination equipment removably connected to the endoscope apparatus 2 and a light source device 4, a light source device 4 that is connected to the endoscope apparatus 2 and the scope 3 and that generates illumination light used by the endoscope 3 to perform an examination, and an image server 5 (server) that is a medical server connected to the endoscope apparatus 2 through the information network 40.

The endoscope 3 is provided, for example, a solid-state image pickup device (CCD) 3a for capturing an image of a subject, at an inner portion of a tip of an insertion part, and a storage device 3b such as an EPROM in which identification information such as a scope ID indicating the type of this endoscope 3 is stored in advance.

The endoscope 3 may be, as illustrated in FIG. 2 for example, a visible light examination endoscope 3A for performing endoscopy with ordinary visible light, an ultrasonographic examination endoscope 3B for performing ultrasonography by using ultrasound, or a special-light examination endoscope 3C for performing special-light endoscopy by using a special light.

As is known in the art, each of such endoscopes 3A-3C is provided with a long slender insertion part 3x that is inserted into a body cavity, a holding unit 3y that is provided on a base end side of the insertion part 3x, and a connector unit 3z that is provided on a base end side of a universal code and is removably attached to the endoscope apparatus 2.

The endoscope 3 that corresponds to one of such endoscopes 3A-3C is selected according to what is to be examined. Then, as illustrated in FIG. 2, the endoscope 3 is connected to the light source device 4 by inserting the connector unit 3z of the selected endoscope 3 into the connector unit 4A of the light source device 4, and the endoscope 3 is connected to the endoscope apparatus 2 through a scope cable (not illustrated).

When the endoscope 3 is connected to the light source device 4 and the endoscope apparatus 2, it is configured such that identification information such as a scope ID that is stored in the storage device 3b of the endoscope 3 will be acquired by a later-described scope interface unit 6 in the endoscope apparatus 2 through a scope cable (not illustrated).

A light guide cable that carries illumination light is arranged in a scope cable that connects the light source device 4 to the endoscope 3. The light source device 4 provides illumination light from the light source device 4 to a light guide of the endoscope 3, and illuminates an affected part or the like in the body cavity of a patient into which the insertion part 3x of the endoscope 3 is inserted.

Then, the endoscope 3 captures an optical image of an affected part or the like by an observation optical system (not illustrated), using a CCD 3a, which is arranged inside a tip portion of the insertion part 3x, and transmits a capture signal, which is obtained by capturing an image, to the endoscope apparatus 2 through a universal code.

The endoscope apparatus 2 stores the capture signal received from the endoscope 3 in the memory 8 as the image data of a medical image.

Next, the configuration of the light source device 4 that supplies illumination light to the endoscope 3 will be described. As illustrated in FIG. 1, the light source device 4 is provided with a communication interface unit 14 for communicating with the endoscope apparatus 2, a lamp 15 that is a light source for generating illumination light, a light amount adjuster 16 for adjusting the amount of light of the lamp 15, a special-light filter controller 17 for controlling a drive of a special-light filter that generates special light when the special-light examination endoscope 3C is connected to the special-light filter controller 17, and a controller 18 for controlling the entire light source device 4 that includes the blocks as above.

The communication interface unit 14 is an interface for communicating with a later-described light source communication interface unit 9 of the endoscope apparatus 2 by establishing an electrical connection therebetween. For example, when the operation of selecting a light source filter is performed, it is configured such that identification information such as the type of the selected light source filter will be transmitted to a light source communication interface unit 9 of the endoscope apparatus 2.

Next, an example of the specific configuration of the endoscope apparatus 2 and image server 5 that compose the medical system 1 according to the present embodiment will be described.

As illustrated in FIG. 1, the endoscope apparatus 2 of the present embodiment is a DICOM-supported digital imaging device, and is provided with the scope interface unit 6, the image generation unit 7, the memory 8, an image processing unit 10, a nonvolatile storage device 11, a network interface unit 12 (communication unit), and a controller 13.

In the present embodiment, the storage device 11 in the endoscope apparatus 2 stores, for example, basic software 11a such as an operating system (OS) that is required to display DICOM image data to be output and an application program 11b (control unit) (first controlunit) that is a control program for realizing the cooperative operation with the DICOM-compatible image server 5.

In the present embodiment, the nonvolatile storage device 11 further stores a later-described modality-side status table 50 (examination status storage unit) (first examination status storage unit) for the management of the examination status in the endoscope apparatus 2.

Moreover, in the present embodiment, the application program 11b is executed by the controller 13 to include control logic to realize the function of access to the modality-side status table 50 as necessary and sending a notification of the examination status in the endoscope apparatus 2 to the image server 5 when, for example, communication is established with the image server 5, and the function of shifting the endoscope apparatus 2 to the termination of the examination according to an examination complete executive instruction from the image server 5, or the like.

The controller 13 is configured, for example, by a microprocessor, and controls the entirety of the endoscope apparatus 2 that composes the medical system 1 by executing the basic software 11a and application program 11b that are stored in the storage device 11.

The scope interface unit 6 is an interface for the endoscope 3 to be removably and electrically connected to the endoscope apparatus 2. When the endoscope 3 is connected to the endoscope apparatus 2, identification information such as a scope ID stored in the storage device 3b of the endoscope 3 is acquired, and the scope interface unit 6 processes the acquired identification information by a scope ID processing unit 6a and then outputs the processed identification information to the controller 13.

The image generation unit 7 acquires a capture signal that is obtained by capturing an image by the CCD 3a of the endoscope 3, and then performs signal processing on the acquired capture signal to output the resultant data to the image processing unit 10 through the controller 13. The memory 8 stores the data of a medical image such as an endoscopic image obtained by the endoscope 3.

The light source communication interface unit 9 is an interface for communicating with the communication interface unit 14 of the light source device 4 by establishing an electrical connection. For example, when the operation of selecting a light source filter of the light source device 4 is performed, identification information such as the type of the selected light source filter is transmitted from the light source device 4, and the light source communication interface unit 9 receives and acquires the transmitted identification information and outputs the acquired information to the controller 18.

The image processing unit 10 processes the image data provided from the image generation unit 7 so as to be output to a connected display device (not illustrated) and to the image server 5 that is connected through the network interface unit 12.

For example, when the image data is output to the image server 5 through the network interface unit 12, the image processing unit 10 performs a specified process on the image data so as to be DICOM-supported image data by the control of the controller 13.

The network interface unit 12 is an interface for communicating information with the image server 5 through the information network 40, and outputs DICOM-supported image data such as an endoscopic image processed by the image processing unit 10 to the image server 5 through the information network 40.

In the present embodiment, the network interface unit 12 also transmits examination status information such as under examination and examination complete of the endoscope apparatus 2 to the image server 5, and receives an examination complete executive instruction from the image server 5.

On the other hand, the image server 5 that composes the medical system 1 according to the present embodiment is provided with a network interface unit 19 (communication unit), an external storage 20, a memory 21, and a CPU 22.

The network interface unit 19 is an interface for communicating information with the endoscope apparatus 2 through the information network 40. In particular, the network interface unit 19 exchanges examination status information such as a later-described examination start and examination complete with the endoscope apparatus 2, or receives DICOM-supported image data such as an endoscopic image generated by the endoscope apparatus 2 through the information network 40.

The external storage 20 is composed of a nonvolatile storage device. In the present embodiment, the external storage 20 includes basic software 20a for controlling the entirety of the image server 5 and an application program 20b (second control unit) (control unit) for realizing cooperative operation or the like with the DICOM-compatible endoscope apparatus 2.

Moreover, the external storage 20 includes a server-side status table 60 (second examination status storage unit) therein for managing the status of the image server 5.

In the present embodiment, the application program 20b is executed by the CPU 22 to realize a function of access to the server-side status table 60 as necessary, and generating an examination complete executive instruction to change from the image server 5 the examination status of the endoscope apparatus 2 according to the examination status information received from the endoscope apparatus 2 and the status of the image server 5.

The CPU 22 controls the entirety of the image server 5 by executing the basic software 20a and the application program 20b loaded onto the memory 21, and realizes the cooperative operation or the like with the DICOM-compatible endoscope apparatus 2 as described above.

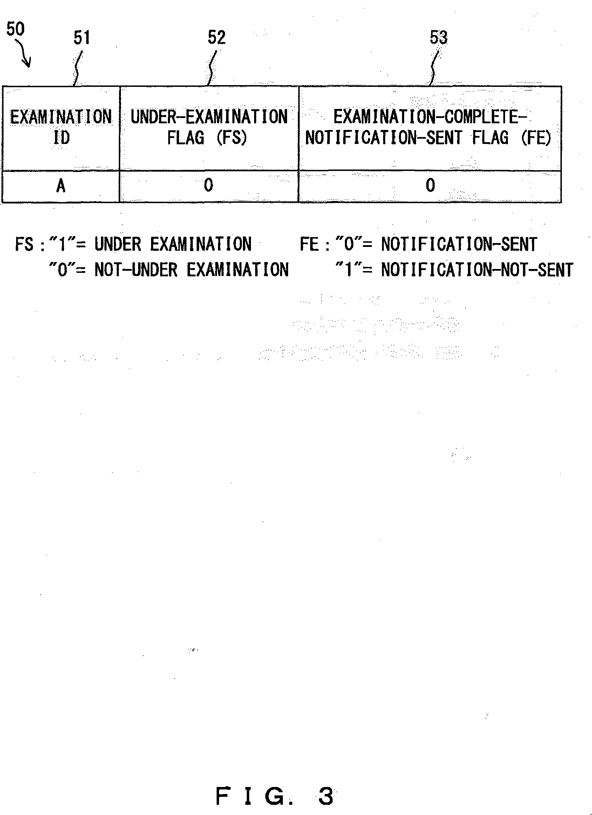

FIG. 3 is a schematic diagram depicting an example of the configuration of the modality-side status table 50 provided in the endoscope apparatus 2 of the present embodiment.

In the modality-side status table 50, examination IDs 51, under-examination flags 52, and examination-complete-notification-sent flags 53 are associated with each other and stored.

The examination ID 51 is information for uniquely identifying each one of the examinations performed by the endoscope apparatus 2.

The under-examination flag 52 is a region in which an under-examination flag FS is stored that indicates whether the endoscope apparatus 2 is under examination or in a state of examination complete (not-under examination), and is set and updated by the application program 11b, where under examination="1" and not-under examination="0".

The examination complete-notification-sent flag 53 is a region in which an examination complete-notification-sent flag FE is stored that manages whether or not the status of the corresponding under-examination flag 52 has been sent to the image server 5, and is set and updated by the application program 11b, where notification-not-sent="1" and notification-sent="0".

In other words, when a notification that communication is established is received from the network interface unit 12 and the under-examination flag FS is "1", indicating an under-examination state, the controller 13 of the endoscope apparatus 2 is able to recognize that such an established communication is a communication that is re-established after the communication is interrupted during examination.

Moreover, when the under-examination flag FS is "0", indicating an examination complete state, and the examination complete-notification-sent flag FE is "1", indicating that a notification has not been sent, it is possible to recognize that such an established communication is a communication that is re-established immediately after an examination ended while communication was being interrupted.

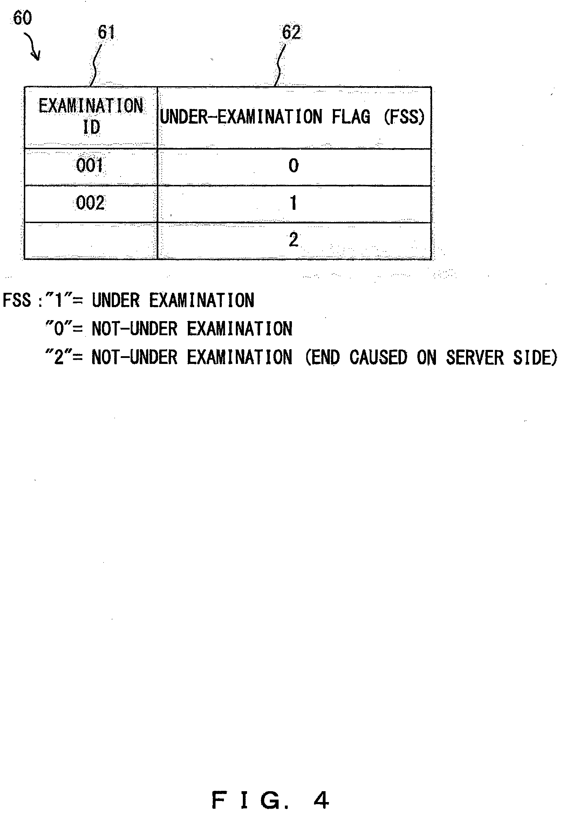

FIG. 4 is a schematic diagram depicting an example of the configuration of the server-side status table 60 provided for the image server 5 of the present embodiment.

In the server-side status table 60, examination IDs 61 and under-examination flags 62 are associated with each other and stored.

The examination ID 61 is information for uniquely identifying the examinations performed by the endoscope apparatuses 2 connected to the image server 5. In other words, the examination performed in any of the endoscope apparatuses 2 may be uniquely distinguished by the examination ID 61.

The under-examination flag 62 (under-examination flag FS_S) is a region in which information is stored that indicates the examination status of the examination specified by the corresponding examination ID 61 from among the examinations performed by the respective endoscope apparatuses 2, and is set and updated by the application program 20b.

In the under-examination flags 62, the information of not-under examination="2" that indicates the not-under-examination state due to the examination status of examination complete caused by an operation on the image server 5 side may be stored in addition to under examination ="1" and not-under examination="0".

In other words, not-under examination="2" on the under-examination flag 62 (under-examination flag FS_S) indicates the not-under-examination state due to the examination complete caused by an operation on the image server 5 side.

Once notification that communication is established is received from the network interface unit 19, and when the under-examination flag FS_S of the server-side status table 60 that corresponds to the examination ID 61 of a desired endoscope apparatus 2 is "1", it is possible for the CPU 22 of the image server 5 to determine that the communication has been re-established in the endoscope apparatus 2 after the communication is interrupted during examination.

When the under-examination flag FS_S is "2", it is possible for the CPU 22 of the image server 5 to determine that the communication has been re-established immediately after examination complete is executed by the operation of the image server 5 while communication has been cut, the cutting having been initiated while the endoscope apparatus 2 was being examined, regardless of the notification from the endoscope apparatus 2.

Hereinafter, an example of the operation of the medical system 1 according to the present embodiment will be explained.

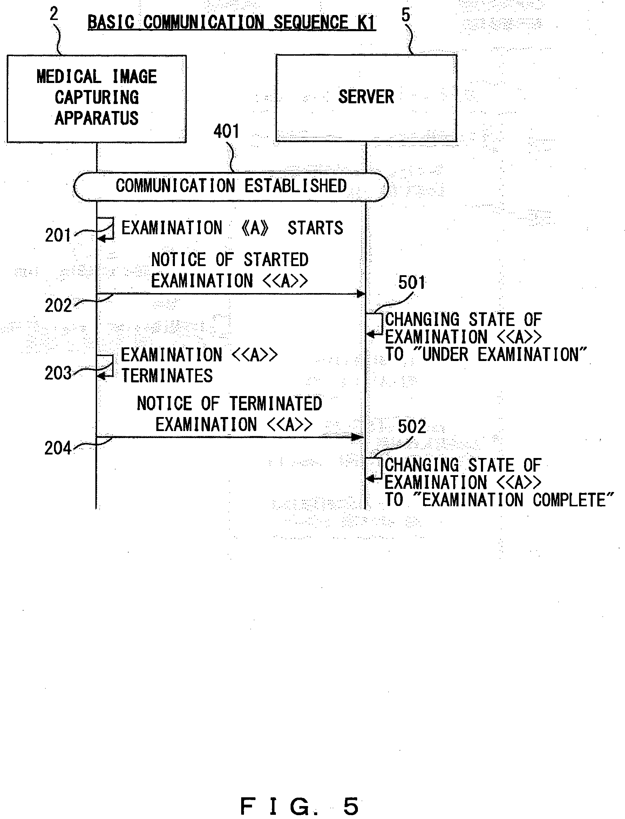

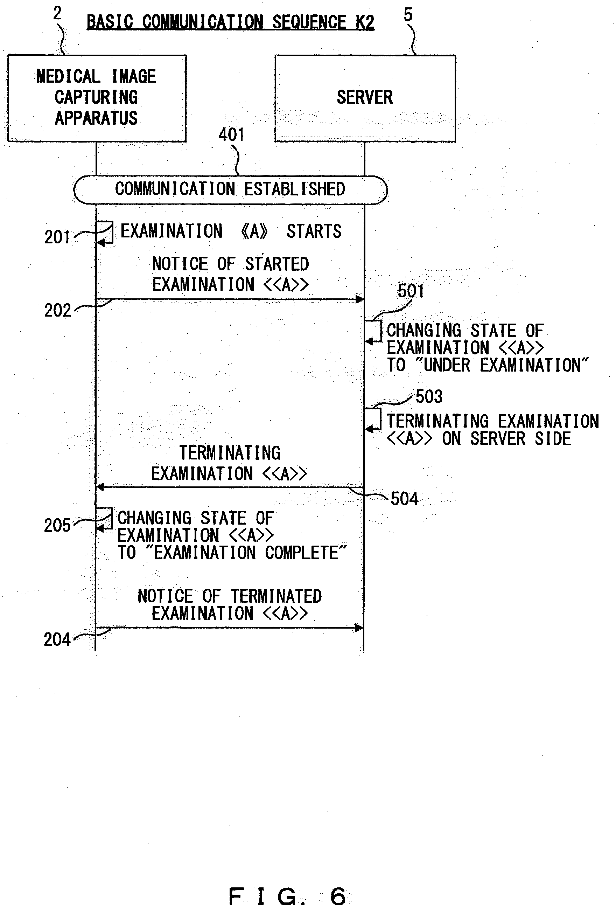

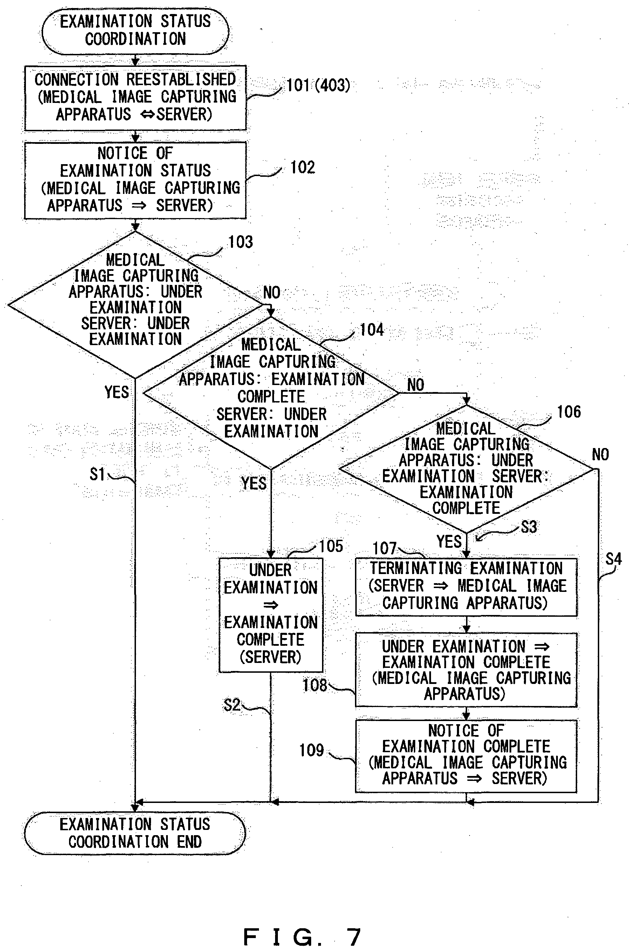

FIG. 5 and FIG. 6 are sequence diagrams depicting a basic communication sequence in the medical system 1 according to the present embodiment. FIG. 7 is a flowchart illustrating an example of the processing in which the states of the endoscope apparatus 2 and the image server 5 are coordinated in the medical system 1 according to the present embodiment.

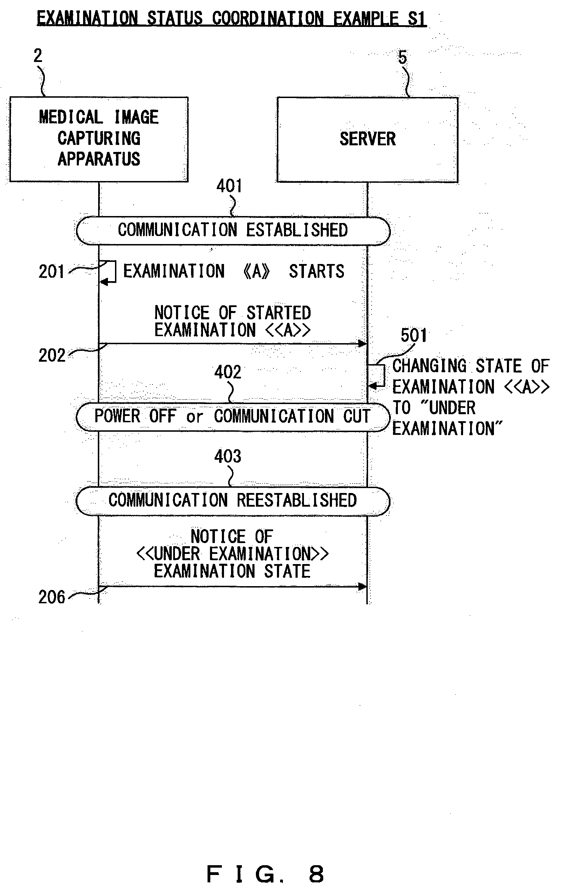

Moreover, FIGs. 8, 9, 10, and 11 are sequence diagrams which each illustrate an example: of the processing in which the states of the endoscope apparatus 2 and the image server 5 are coordinated in the medical system 1 according to the present embodiment.

Firstly, an example of the operation of the endoscope apparatus 2 and the image server 5 under normal conditions in which communication is not interrupted between the image server 5 and the endoscope apparatus 2 while one examination starts and ends will be explained with reference to FIG. 5 and FIG. 6.

FIG. 5 illustrates an example in which the state of the image server 5 is coordinated with the change in examination status of the endoscope apparatus 2 by tracking the examination status of the endoscope apparatus 2 (basic communication sequence K1).

In other words, when the communication between the endoscope apparatus 2 and the image server 5 is established, the controller 13 that executes the application program 11b via the network interface unit 12 (hereinafter, referred to simply as controller 13) in the endoscope apparatus 2 is notified of the established communication.

Subsequently, a specified examination in which the endoscope 3 is used is started in the endoscope apparatus 2, and the controller 13 detects the specified examination and sets examination identification information (in this case, examination A) to the examination ID 51 in the modality-side status table 50 and sets "1" indicating under examination to the under-examination flag 52 as the under-examination flag FS. Further, the controller 13 sets "1" indicating notification-not-sent to the examination complete-notification-sent flag 53 as the examination complete-notification-sent flag FE (step 201).

Subsequently, the controller 13 of the endoscope apparatus 2 notifies the image server 5 of the started examination A (step 202 (first step)).

On the other hand, the CPU 22 that executes the application program 20b via the network interface unit 19 upon the establishment of communication above (hereinafter, referred to simply as the CPU 22) is notified of the established communication on the image server 5 side.

Then, when the CPU 22 receives from the endoscope apparatus 2 the above notification of the started examination A, the CPU 22 sets the identification information of the endoscope apparatus 2 to the examination ID 61 of the server-side status table 60 and sets "1" indicating under examination to the corresponding under-examination flag 62 as the under-examination flag FS_S, thereby changing the state of the image server 5 to under examination (step 501).

When the examination A ends on the endoscope apparatus 2 side (step 203), the controller 13 transfers medical images generated in the endoscope apparatus 2 during the examination A to the image server 5 and notifies the image server 5 of the termination of the examination A, and changes the under-examination flag 52 on the modality-side status table 50 and the examination complete-notification-sent flag 53 to not-under examination="0" and "0", respectively (step 204).

Then, the CPU 22 of the image server 5 stores medical images of the examination A received from the endoscope apparatus 2 in the external storage 20 or the like, and changes the under-examination flag 62 on the server-side status table 60 to not-under examination="0" so as to change the state of the image server 5 to examination complete (step 502 (third step)).

By so doing, control is performed such that the examination status of the endoscope apparatus 2 and the image server 5 will be coordinated with each other.

FIG. 6 illustrates an example in which the image server 5 forces the endoscope apparatus 2 to shift from under examination to examination complete when necessary (basic communication sequence K2).

In such cases, when a user requests that the examination by the endoscope apparatus 2 on the image server 5 side be terminated after the state is changed to under examination as in step 501 above, the CPU 22 sets the under-examination flag 62 on the server-side status table 60 to not-under examination="2" indicating examination complete due to the operation on the image server 5 side to terminate the examination A on the image server 5 side by the operation on the image server 5 side (step 503), and notifies the endoscope apparatus 2 of an instruction to terminate the examination A (step 504).

Note that in step 503, not-under examination="2" indicating examination complete due to the operation on the image server 5 side is set as the under-examination flag 62. This feature is for distinguishing whether the state of not-under examination on the image server 5 is caused by the notification from the endoscope apparatus 2 or by the examination complete due to the operation on the image server 5 side when communication is re-established, as will be described later.

Then, the controller 13 of the endoscope apparatus 2 terminates the examination A and changes the under-examination flag 52 on the modality-side status table 50 to not-under examination="0" (step 205), and further notifies the image server 5 of the termination of the examination A (step 204).

As described above, even when a request is made for an examination complete due to the operation on the image server 5 side, it is controlled such that the examination status of both the endoscope apparatus 2 and the image server 5 will be coordinated with each other.

Next, in the medical system 1 according to the present embodiment, processes caused by interrupted communication and re-established communication between the endoscope apparatus 2 and the image server 5 will be explained.

As illustrated in the flowchart of FIG. 7, once the communication between the endoscope apparatus 2 and the image server 5 is re-established (step 101), the controller 13 of the endoscope apparatus 2 notifies the image server 5 of the examination status by referring to the under-examination flag 52 on the modality-side status table 50 (step 102).

When the examination status of both the endoscope apparatus 2 and the image server 5 is under examination ("YES" in step 103), as illustrated in FIG. 8 as examination status coordination example S1, the examination by the endoscope apparatus 2 is continued just as it is as long as the endoscope apparatus 2 sends a notice of being under examination as the state of the examination A (step 206).

On the other hand, when step 103 is "NO" and the image server 5 and the endoscope apparatus 2 are in the state of under examination and examination complete (not-under examination), respectively ("YES" in step 104), the state of the image server 5 shifts from under examination to examination complete (step 105).

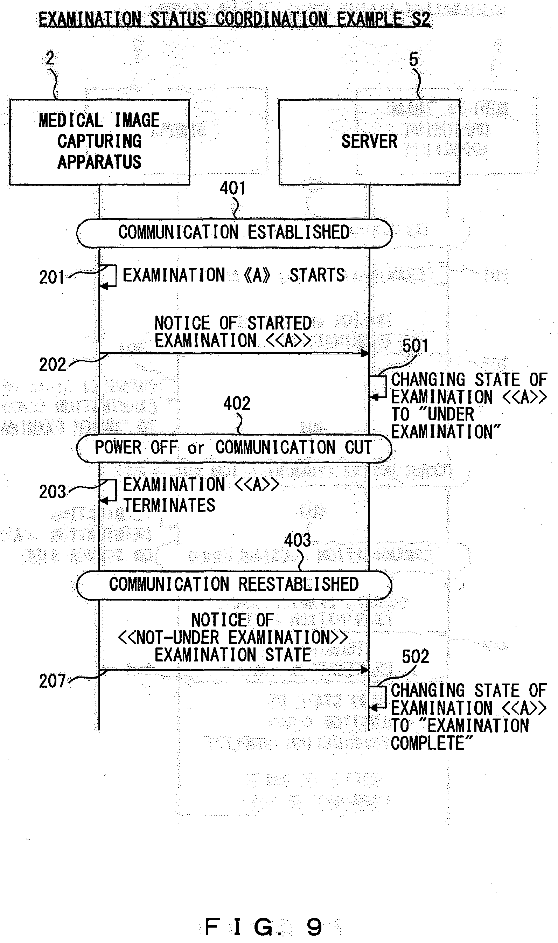

In other words, in such cases, as illustrated in FIG. 9 as examination status coordination example S2, the endoscope apparatus 2 terminates the examination A while communication is being interrupted and before communication is re-established (step 403), and the endoscope apparatus 2 notifies the image server 5 of the examination status of not-under examination (step 207 (second step). In the image server 5, the CPU 22 changes the under-examination flag 62 on the server-side status table 60 to not-under examination="0" (step 502).

By so doing, even when the state of examination complete is caused on the endoscope apparatus 2 side while communication is being interrupted, it becomes possible to coordinate the examination status of the endoscope apparatus 2 and the image server 5.

When step 104 of FIG. 7 is "NO" and the endoscope apparatus 2 and the image server 5 are in the state of under examination and examination complete (not-under examination), respectively ("YES" in step 106), the image server 5 instructs the endoscope apparatus 2 to terminate the examination (step 107), and the endoscope apparatus 2 changes the state from under examination to examination complete (step 108). Then, the endoscope apparatus 2 sends a notice of examination complete to the image server 5 (step 109).

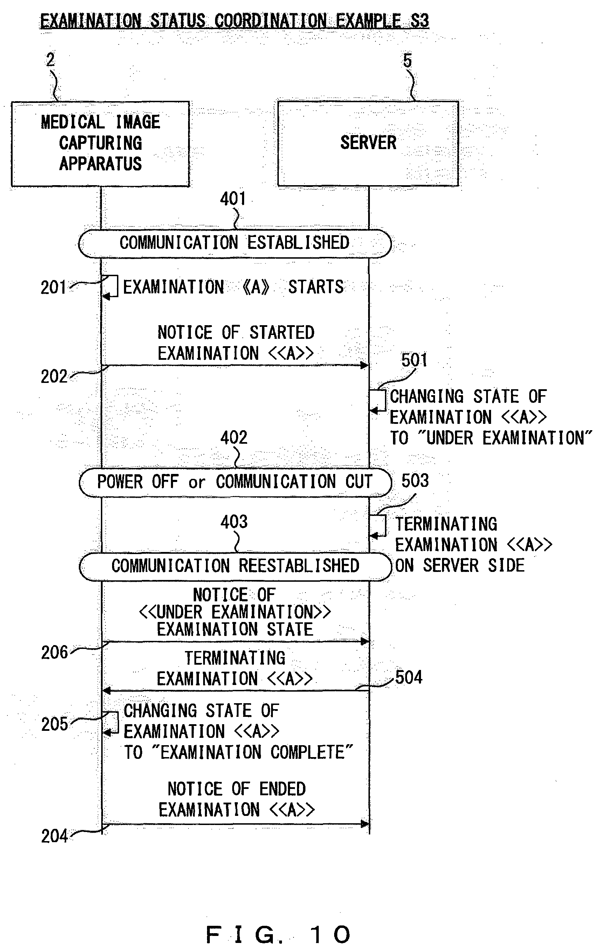

Such a case is equivalent to examination status coordination example S3 of FIG. 10 in which the examination is terminated due to the operation on the image server 5 side while communication is being interrupted (step 503). In this case, when the endoscope apparatus 2 sends a notice of under examination to the image server 5 as the examination status (step 206) after communication is re-established (step 403), the CPU 22 of the image server 5 recognizes that the examination was terminated due to the operation on the image server 5 side because the under-examination flag 62 on the server-side status table 60 is set to not-under examination="2", and the CPU 22 instructs the endoscope apparatus 2 to terminate the currently-running examination A (step 504). In response to this instruction, the controller 13 of the endoscope apparatus 2 changes the under-examination flag 52 on the modality-side status table 50 to not-under examination="0" so as to change the state to examination complete (step 205), and then notifies the image server 5 of the termination of the examination A (step 204).

By so doing, even when a request is made for an examination complete due to the operation on the image server 5 side while communication is being interrupted, it becomes possible to coordinate the examination status of the endoscope apparatus 2 and the image server 5.

"NO" in step 106 of FIG. 7 indicates that the examination has been terminated in both the endoscope apparatus 2 and the image server 5 while communication is being interrupted.

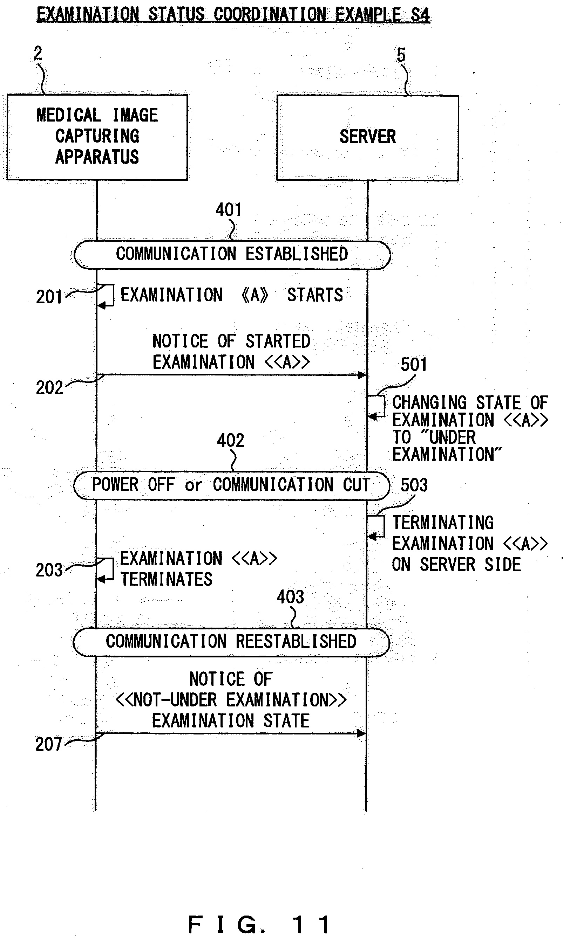

This case is equivalent to an examination status coordination example S4 of FIG. 11, and after communication is re-established (step 403), the endoscope apparatus 2 sends a notice of not-under examination to the image server 5. As the examination has been terminated and the image server 5 is also in the state of not-under examination, there is no contradiction between the two states and thus the process just terminates.

As described above, in the medical system 1 according to the present embodiment, even when communication re-established after the communication via the information network 40 is interrupted as the power source of the endoscope apparatus 2 is cut off, for example due to the under-examination replacement of the endoscope 3 in the endoscope apparatus 2, the coordination of the examination status is maintained between the endoscope apparatus 2 and the image server 5. Accordingly, it becomes possible to accurately recognize, in common between the endoscope apparatus 2 and the image server 5, examination information generated in a single examination, such as medical images, as examination information about the single examination.

As a result, it becomes not necessary for a user, for example, to gather several pieces of examination information that have been split up due to the interrupted communication, which could be a complicated operation. Instead, it becomes possible for a user to manage a series of pieces of examination information about a single examination in a simple and reliable manner.

### (Embodiment 2)

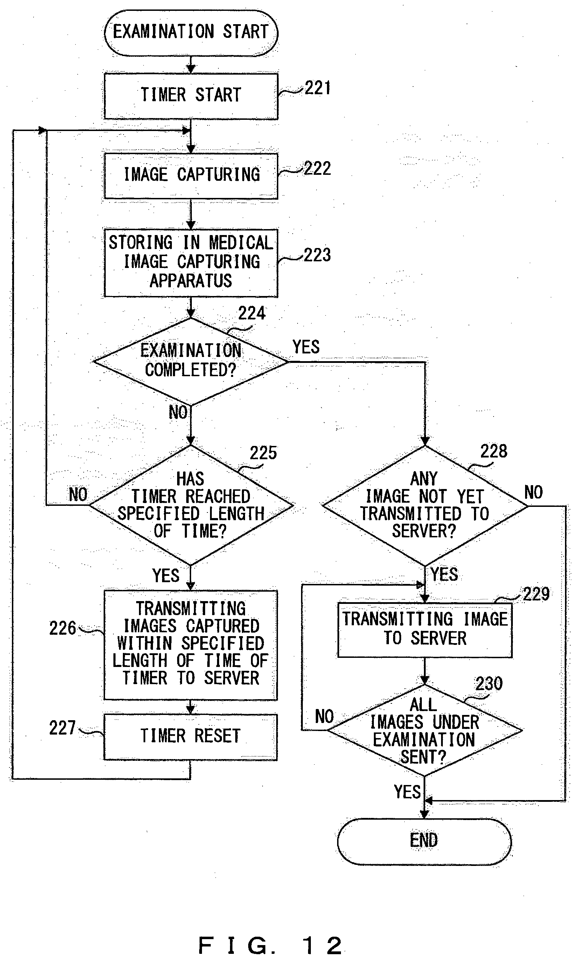

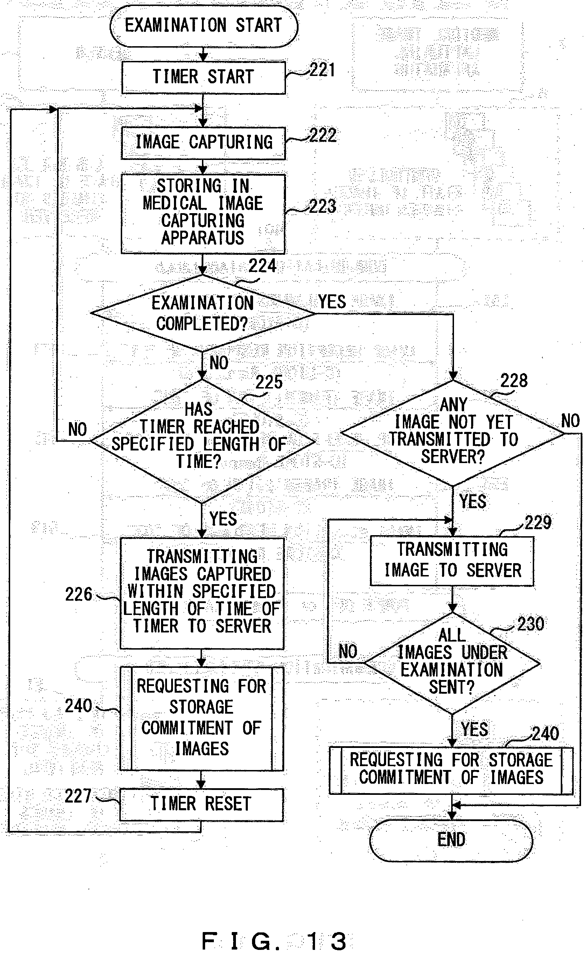

FIG. 12 and FIG. 13 are flowcharts of an example of the operation of the endoscope apparatus 2 in a medical system according to another embodiment of the present invention.

As described in relation to step 204 above, medical images generated at the endoscope apparatus 2 during the examination may be transferred to the image server 5 all at one time when the examination is terminated.

However, in a medical image capturing apparatus such as the endoscope apparatus 2, the amount of medical image data generated during the examination is relatively large. For this reason, if a method is adopted in which the data is transferred to the image server 5 all at one time when the examination is terminated, the length of time required to transfer the data tends to be long. Accordingly, there have been some cases in which it was difficult for the image server 5 to generate a diagnostic report in a prompt manner after the examination was terminated.

To address this problem, in relation to the medical system 1 according to Embodiment 2, cases in which the endoscope apparatus 2 of the above medical system 1 transfers examination information such as medical images during the examination to the image server 5 at specified time intervals during the examination will be described as examples.

Note that the system configuration is shared between the above Embodiment 1 and the present Embodiment 2, and thus overlapping descriptions will be omitted.

In Embodiment 2, the application program 20b is executed by the controller 13, and later-described control functions as depicted in the flowcharts of FIG. 12 and FIG. 13 in addition to the control function described in Embodiment 1 above will be achieved.

In other words, once an examination is started, the controller 13 that executes the application program 20b of the endoscope apparatus 2 in Embodiment 2 starts a timer implemented on the controller 13 for measuring the time (step 221).

Then, the controller 13 performs desired image capturing by using the endoscope 3 (step 222), and stores the captured images in the memory 8 of the endoscope apparatus 2 (step 223).

Then, the controller 13 determines whether or not the examination is completed (step 224). When the examination is not completed, the controller 13 further determines whether or not the value measured by the timer has reached a specified length of time (step 225), and when it is determined that the value has not been reached, step 222 of image capturing and the following steps above are repeated.

On the other hand, when it is determined in step 225 that the value measured by the timer has reached a specified length of time, examination information such as medical images captured during the examination within the specified length of time is transferred to the image server 5 (step 226), and then the timer is reset (step 227) and the process returns to the above-mentioned step 222.

When it is determined in the above-mentioned step 224 that the examination has been completed, the controller 13 determines whether or not there is any medical image that has not been transferred to the image server 5 in the memory 8 (step 228), and if it is determined that there are such medical images, all of those medical images are transferred to the image server 5 (step 229, step 230).

As described above, in the present Embodiment 2, examination information such as medical images that are generated during the examination is transferred to the image server 5 on a regular basis, and thus it becomes possible to shorten the length of time taken to transfer all the images under examination to the image server 5 after the examination is terminated.

As a result, the image server 5 has an advantage that, for example, it becomes possible for the image server 5 to start creating a diagnostic report in a prompt manner after the examination is terminated, and the convenience of the medical system 1 is improved.

FIG. 13 is a flowchart illustrating an example of the modification of the present Embodiment 2. The DICOM standard with which the medical system 1 according to the present Embodiment 2 complies has a function called "storage commitment", where the image server 5 ensures, for the endoscope apparatus 2, the storage of examination information such as medical images received at the image server 5 from the endoscope apparatus 2.

For this purpose, in an example of the modification illustrated in FIG. 13, the endoscope apparatus 2 requests storage commitment of images in the control described above with reference to FIG. 12 for the under-examination transmission of medical images transferred from the endoscope apparatus 2 to the image server 5 on a regular basis (step 240).

In the process of requesting a storage commitment in step 240, the endoscope apparatus 2 sends to the image server 5 a message requesting a storage commitment, and after the reception (storage) by the image server 5 is confirmed, medical images whose requests for a storage commitment are completed, the medical images being stored in the memory 8 within the endoscope apparatus 2, are deleted.

By so doing, it becomes no longer necessary to store, in the memory 8 within the endoscope apparatus 2, medical images whose requests for storage commitment are completed after those medical images are transferred from the endoscope apparatus 2 to the image server 5. Accordingly, in addition to the advantageous effects achieved in the case of the flowchart of FIG. 12 described above, it becomes possible to reduce the cost of the endoscope apparatus 2 as the capacity of the memory 8 is reduced.

### (Embodiment 3)

If communication interruption and communication reestablishment occurs between the endoscope apparatus 2 and the image server 5 during the process of requesting storage commitment described in the Embodiment 2 above, there are concerns about inconsistencies in the controlled state of images between the endoscope apparatus 2 and the image server 5.

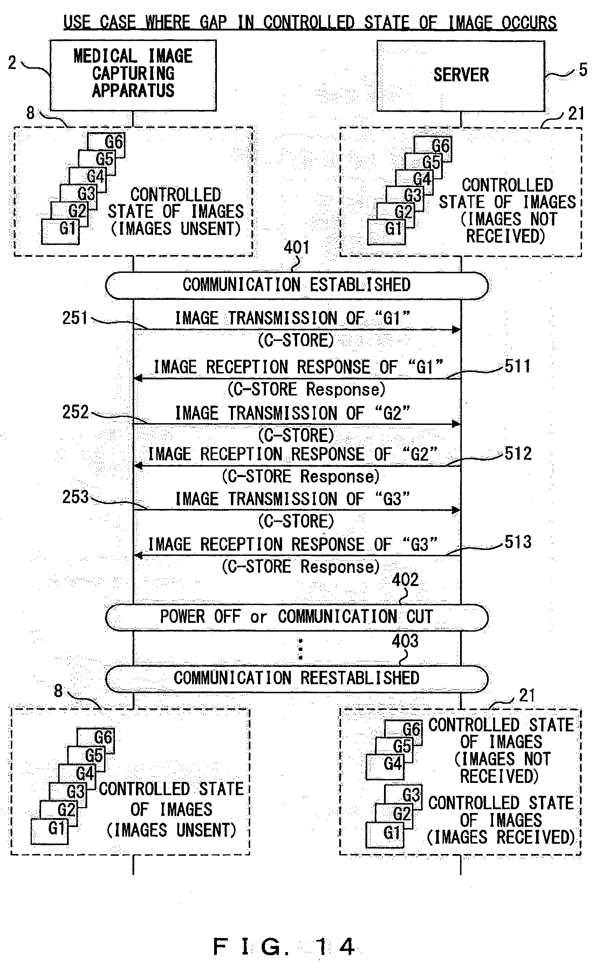

FIG. 14 is a sequence diagram illustrating an example case in which inconsistencies in the controlled state of images are caused by the communication interruption between the endoscope apparatus 2 and the image server 5.

In regard to the processes common to those in the sequence diagrams described above, the same step numerals are given.

As illustrated in FIG. 14 for example, it is assumed that six images of image data G1-G6 are stored in the memory 8 of the endoscope apparatus 2 without being sent to the image server 5.

It is further assumed that after the communication between the endoscope apparatus 2 and the image server 5 is established (step 401), communication is cut (step 402) when image transmission (C-STORE) and image reception response (C-STORE Response) are sequentially repeated for image data G1 through image data G3, and the communication is reestablished later (step 403).

In this case, the endoscope apparatus 2 has not completed the storage commitment for the image server 5, and thus the controlled state of all of the image data G1 through image data G6 (medical image) is still marked as unsent.

On the other hand, the image server 5 has returned a reception response (C-STORE Response) to the endoscope apparatus 2 in regard to the images of "G1" through "G3", and thus the controlled state in the image server 5 is marked as received in regard to the images of "G1" through "G3".

In such a state, the endoscope apparatus 2 tries to perform image transmission (C-STORE) of image data G1 through image data G5 to the image server 5 after the communication is reestablished in step 403. However, the image server 5 has already received the image data G1 through image data G3 as described above, and thus the image server 5 sends a response of rejected reception (C-STORE Response).

There are concerns about operational failures in which the endoscope apparatus 2 further performs retrying of image transmission (C-STORE) a certain number of times in the above situation, and the endoscope apparatus 2 finally stops working after sending an error notice or the like as an occurence of transmission error.

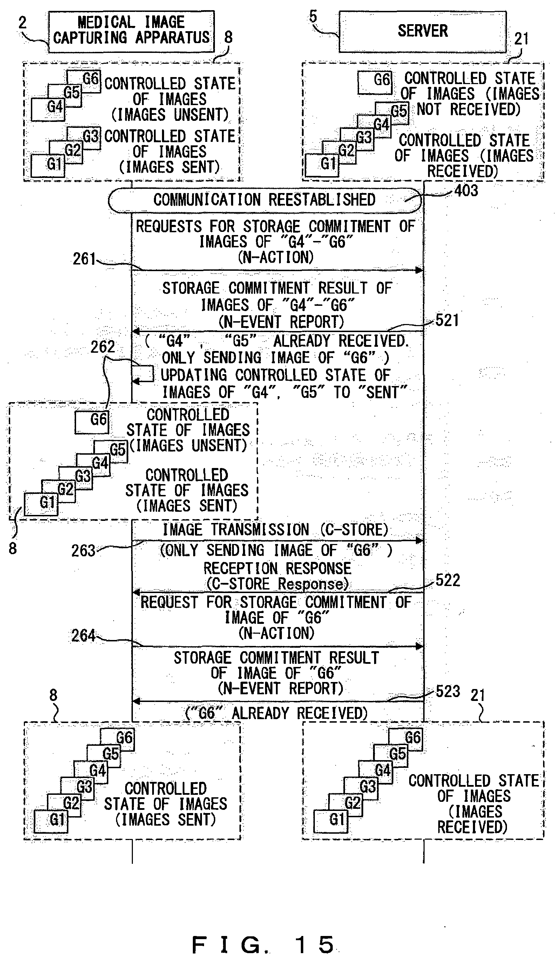

For this reason, in the endoscope apparatus 2 according to the present Embodiment 3, a function is implemented in the application program 11b that prevents a failure due to inconsistencies in the controlled state of images between the endoscope apparatus 2 and the image server 5 caused by communication cutting/communication reestablishment as described above, achieved by the processes of FIG. 15 described below. This function is realized as the controller 13 performs the application program 11b.

In other words, FIG. 15 is a flowchart illustrating an example of the processes performed when the communication is reestablished in the endoscope apparatus 2 of the present Embodiment 3.

In an example of FIG. 15, it is assumed that the endoscope apparatus 2 is in a controlled state where image data G1 through image data G3 from among image data G1 through image data G6 have been sent and the remaining image data G4 through image data G6 have not been sent yet, and that the image server 5 is in a controlled state where image data G1 through image data G5 have already been received and only the image data G6 has not been received yet, at the time immediately before the communication is reestablished (step 403).

When the communication is reestablished between the endoscope apparatus 2 and the image server 5 after the communication is cut (step 403), the controller 13 that executes the application program 11b in the endoscope apparatus 2 of the present embodiment (hereinafter, referred to simply as controller 13) firstly sends requests for a storage commitment of images (N-ACTION) to the image server 5 in regard to image data G4 through image data G6 that are recognized as unsent in the endoscope apparatus 2 (step 261).

In response to the above, the image server 5 sends to the endoscope apparatus 2 a storage commitment result (N-EVENT REPORT) of images in the image data G4 through image data G6 (step 521).

In this case, the current controlled state of images at the image server 5 in which the image data G4 and the image data G5 have been received and only the image data G6 has not yet been sent is sent to the endoscope apparatus 2 by using the storage commitment result (N-EVENT REPORT).

The controller 13 of the endoscope apparatus 2 receives the storage commitment result, and updates the controlled state of the image data G4 and the image data G5 to "sent" in the controlled state of images in the memory 8 (step 262).

Subsequently, the controller 13 of the endoscope apparatus 2 performs image transmission (C-STORE) in which only the unsent image data G6 is sent to the image server 5 (step 263), and the image server 5 sends a reception response (C-STORE Response) by return (step 522).

Subsequently, the controller 13 of the endoscope apparatus 2 performs storage commitment of images (N-ACTION) of the sent image data G6 (step 264), and the image server 5 sends to the endoscope apparatus 2 by return a storage commitment result (N-EVENT REPORT) of the image data G6 indicating that the image data G6 has been stored (step 523).

As a result, the controlled state of images of the image data G1 through image data G6 in the memory 8 of the endoscope apparatus 2 and the memory 21 of the image server 5 is coordinated to a state where all the image data G1 through image data G6 have been sent from the endoscope apparatus 2 to the image server 5.

As described above, the application program 11b in the endoscope apparatus 2 of the present embodiment is executed by the controller 13 when the communication is reestablished after communication interruption so as to implement the following functions of: selectively sending to the image server 5 a storage commitment of image data G4 through image data G6 that are recognized as "unsent" in the controlled state in the memory 8 of the endoscope apparatus 2; matching the controlled state of images in the memory 8 of the endoscope apparatus 2 to the controlled state of images in the memory 21 of the image server 5 according to a storage commitment result sent from the image server 5 by return; and selectively sending the image data that is determined to be "unsent" in the memory 8 after the above matching process to the image server 5.

Accordingly, even if a recognition gap in the controlled state of the transmission of image data occurs between the endoscope apparatus 2 and the image server 5 due to communication cutting or the like that occurred while an image was being transmitted from the endoscope apparatus 2 to the image server 5, it becomes possible to control the communication traffic in the information network 40 without causing a retry in the communication of image data between the endoscope apparatus 2 and the image server 5. This is because both of the controlled states of the transmission are coordinated with no contradiction and then only necessary image data is selectively sent from the endoscope apparatus 2 to the image server 5.

In other words, a failure in which unnecessary data transmission is repeated in error may be prevented from occurring in regard to the image data that is managed as "received" in the memory 21 on the image server 5 side.

As a result, a rate at which the medical system 1 shuts down due to a failure is reduced, and the availability of the medical system 1 improves. Moreover, it becomes possible to prevent the communication load on the information network 40 due to the repeated transmission of unnecessary image data from increasing, and the information network 40 may be used in an efficient manner.

In other words, when the communication is reestablished after communication interruption, it becomes possible to efficiently resolve the above-mentioned inconsistencies in the controlled state of images in requesting storage commitment of the medical images from the endoscope apparatus 2 to the image server 5, and smooth operation of the medical system 1 may be achieved.

### (Reference Example 1)

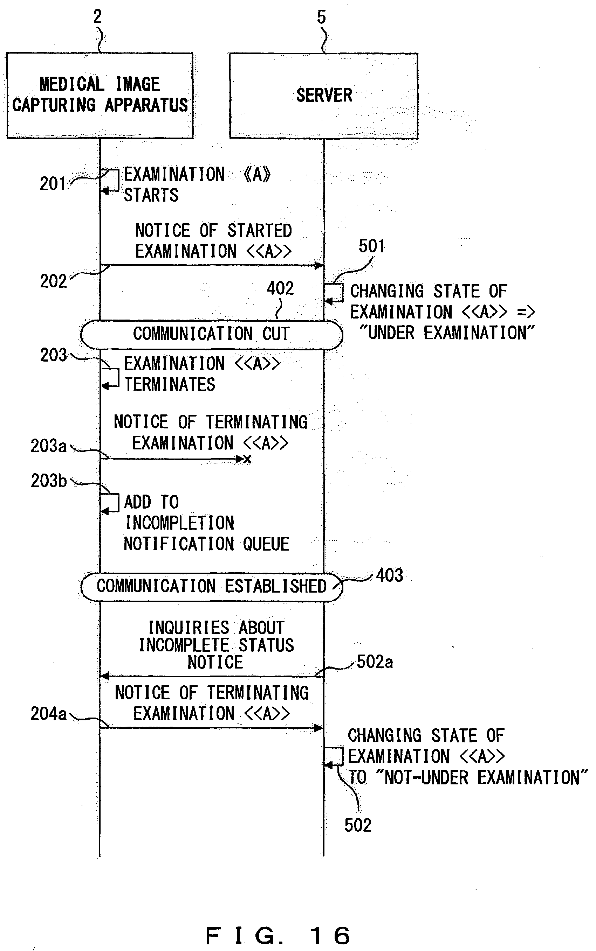

FIG. 16 is a sequence diagram illustrating a reference example that relates to the above-mentioned Embodiment 1. In regard to the processing steps common to those in Embodiment 1 described above, the same reference signs are given, and overlapping descriptions will be omitted.

When a large number of endoscope apparatuses 2 are connected to a single image server 5 in Embodiment 1 described above, there is a possibility that the image server 5 will receive notices of examination status from a large number of endoscope apparatuses 2 at the same time as when the communication is established, and that the image server 5 will be forced to perform the examination status matching process with this large number of endoscope apparatuses 2 all at once within a short period of time. In such a case, the processing load on the image server 5 will be increased, and this may lead to the degradation or failure of the medical system 1.

For this reason, in Reference Example 1, the notices sent while the communication is being cut are accumulated in an incompletion notification queue (not illustrated) (for example, set to the memory 8) in each of the endoscope apparatuses 2 so as to prevent the load on the image server 5 from increasing and to reduce the processing load on the image server 5.

After the communication is reestablished with the individual endoscope apparatuses 2, the image server 5 sequentially sends inquiries to the individual endoscope apparatuses 2. After inquiries are received from the image server 5, the individual endoscope apparatuses 2 send to the image server 5 the uncompleted examination complete notice/examination start notices accumulated in the incompletion notification queue of the individual endoscope apparatus 2.

Accordingly, the image server 5 may take the initiative in collecting the examination status information in the individual endoscope apparatuses 2, and the matching between the image server 5 and the endoscope apparatuses 2 may be precisely performed without increasing the load on the image server 5.

In other words, examination complete (step 203) occurs at the endoscope apparatus 2 between communication cutting (step 402) and communication reestablishment (step 403) as illustrated in FIG. 16, and when the transmission of an examination complete notice to the image server 5 fails due to communication interruption (step 203a), the failed status notice is additionally accumulated in the incompletion notification queue (step 203b).

After the communication is reestablished (step 403), the image server 5 sequentially sends inquiries to each of the connected endoscope apparatuses 2 about an incomplete status notice (step 502a).

In response to the above, the endoscope apparatus 2 sends the status notice stored in the incompletion notification queue (in the case of FIG. 16, the complete notice of Examination A) to the image server 5 (step 204a).

In response to the above, the image server 5 changes the examination status within the image server 5 to "examination complete" (step 502).

After step 502 above, the image server 5 sends inquiries to another one of the connected endoscope apparatuses 2 about incomplete status notices (step 502a).

By so doing, it becomes possible to securely prevent the load on the image server 5 from increasing due to a flood of status notices sent from the several endoscope apparatuses 2 to the image server 5 when the communication is reestablished (step 403).

As a result, it becomes possible to prevent the processing load on the image server 5 from increasing, and the degradation or failure of the medical system 1 may be prevented.

### (Reference Example 2)

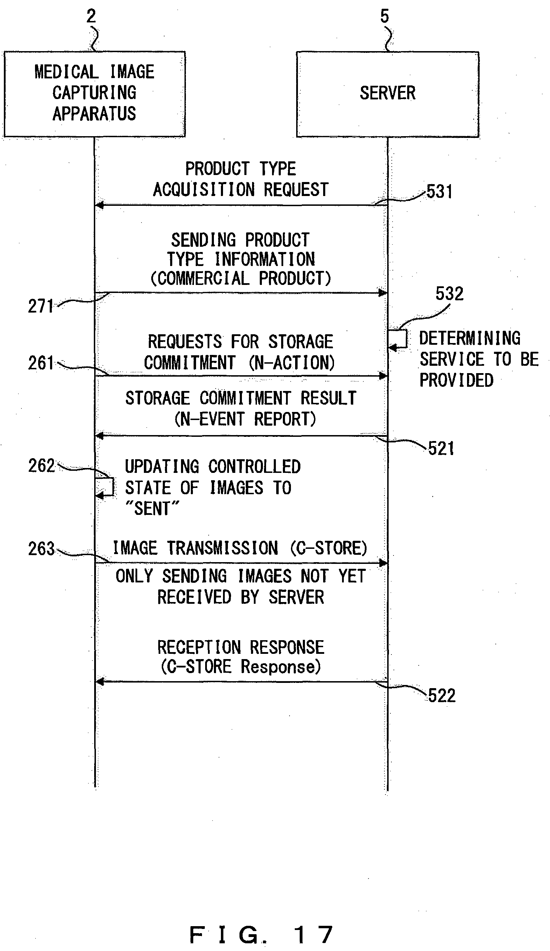

FIG. 17 is a sequence diagram illustrating a reference example that is related to Embodiment 3 above. In regard to the processing steps common to those in Embodiment 3 described above, the same reference signs are given, and overlapping descriptions will be omitted.

In Reference Example 2, an example is described where the image server 5 acquires the product type information (commercial product/trial product/substitute for product under repair, etc.) held in the endoscope apparatus 2 when the image server 5 is connected to the endoscope apparatus 2 prior to the processing in which inconsistencies in the controlled state of images between the endoscope apparatus 2 and the image server 5 are resolved as described in Embodiment 3 above, the image server 5 sorts out services to be provided to the endoscope apparatus 2 according to the acquired information, and the resource management of the endoscope apparatus 2 is performed on the image server 5 side.

In other words, as depicted in FIG. 17, requests for storage commitment of images are sent prior to the transmission of the images. Further, prior to the processes described above in which the controlled state of images on both sides is matched, the image server 5 transmits a product type acquisition request to the endoscope apparatus 2 (step 531), and in response to this transmission, the endoscope apparatus 2 sends product type information or the like to the image server 5 (step 271).

The image server 5 determines the service to be provided to the endoscope apparatus 2 according to the product type information or the like received from the endoscope apparatus 2 (step 532).

Note that the present invention is not limited to the configuration described in the above embodiments, but various modifications and variations may be made by those skilled in the art.

### (Appendix 1)

A medical system including a medical image capturing apparatus and a server, the medical image capturing apparatus comprising:
communication unit for transmitting and receiving data between the medical image capturing apparatus and the server;
examination status notification unit for sending an examination status from the medical image capturing apparatus to the server;
examination complete execution unit for ending an examination of the medical image capturing apparatus by the server; and
control unit for performing matching of an examination status between the medical image capturing apparatus and the server by using the examination status notification unit and the examination complete execution unit.

### (Appendix 2)

A medical image management system including a DICOM-compatible medical image capturing apparatus and an image storage server, the medical image capturing apparatus comprising:
communication unit for transmitting and receiving data
   between the medical image capturing apparatus and the image storage server;
identification unit for identifying an examination status; and
image transmission control unit for transmitting an image at regular intervals during an examination.

### (Appendix 3)

A medical image management system including a DICOM-compatible medical image capturing apparatus and an image storage server, the medical image capturing apparatus comprising:
communication unit for transmitting and receiving data between the medical image capturing apparatus and the image storage server;
check unit for checking a controlled state of images in a server; and
control unit for using the check unit to check the controlled state of images in the server prior to image transmission.

### [Explanation of Reference Signs]

1 medical system
2 endoscope apparatus
3 endoscope
3A visible light examination endoscope 3A
3B ultrasonographic examination endoscope
3C special-light examination endoscope
3a solid-state image pickup device (CCD)
3b storage device
3x insertion part
3y holding unit
3z connector unit
4 light source device
4A connector unit
5 image server
6 scope interface unit
6a scope ID processing unit
7 image generation unit
8 memory
9 light source communication interface unit
10 image processing unit
11 storage device
11a basic software
11b application program
12 network interface unit
13 controller
14 communication interface unit
15 lamp
16 light amount adjuster
17 special-light filter controller
18 controller
19 network interface unit
20 external storage
20a basic software
20b application program
21 memory
22 CPU
40 information network
50 modality-side status table
51 examination ID
52 under-examination flag
53 examination-complete-notification-sent flag
60 server-side status table
61 examination ID
62 under-examination flag
G1-G6 image data
FS under-examination flag on endoscope apparatus 2 side
FE examination-complete-notification-sent flag on endoscope apparatus 2 side
FS_S under-examination flag on image server 5 side

## Claims

1. A medical system (1) comprising a medical image capturing apparatus (2) and a server (5) configured for recording a medical image transmitted from the medical image capturing apparatus (2), the medical system comprising:
a first examination status storage unit (50) of the medical image capturing apparatus (2) configured for storing an examination status of under examination or examination complete;
a second examination status storage unit (60) of the server (5) configured for storing an examination status of under examination or examination complete;
a first control unit (13) of the medical image capturing apparatus (2) configured for notifying the server (5) of the examination status stored in the first examination status storage unit (50) in the medical image capturing apparatus (2);
a second control unit (22) of the server (5) configured for generating an examination complete executive instruction that triggers the medical image capturing apparatus (2) to change the examination status of the medical image capturing apparatus (2) so that the examination status of the medical image capturing apparatus (2) stored in the first examination status storage unit (50) and the examination status of the server (5) stored in the second examination status storage unit (60) correspond,
wherein, when communication reestablishment after communication with the server (5) is cut is detected by the first control unit (13), and when an under examination status is stored in the first examination status storage unit (50) and an examination complete status is stored in the second examination status storage unit (60):
the second control unit (22) is configured to instruct the medical image capturing apparatus (2) to terminate the currently running examination, and
in response to the instruction from the second control unit (22), the first control unit (13) of the medical image capturing apparatus (2) is configured to change the state of the medical image capturing apparatus (2) stored in the first examination status storage unit (50) to examination complete.

2. The medical system according to claim 1, wherein the first control unit (13) is further configured for transmitting the medical image acquired by the medical image capturing apparatus (2) to the server (5) at specified time intervals while under examination.

3. The medical system according to claim 1, wherein the first control unit (13) is further configured for checking a controlled state of the medical image in the server side by sending a request for storage of an unsent medical image of the medical image capturing apparatus (2) to the server (5) when communication is reestablished after a communication interruption between the medical image capturing apparatus (2) and the server (5), and selectively transmitting the medical image which is unsent to the server (5).

4. A communication control method for a medical system (1) comprising a medical image capturing apparatus (2) having a first control unit (13) and a server (5) having a second control unit (22), wherein the medical image capturing apparatus (2) has a first examination status storage unit (50) for storing an examination status of under examination or examination complete, wherein the server (5) has a second examination status storage unit (60) for storing an examination status of under examination or examination complete, and wherein the server (5) records a medical image transmitted from the medical image capturing apparatus (2), the method comprising:
when communication reestablishment after communication with the server (5) is cut is detected by the first control unit (13), and when an under examination status is stored in the first examination status storage unit (50) and an examination complete status is stored in the second examination status storage unit (60):
notifying (206), by the first control unit (13), the server (5) of the examination status stored in the first examination status storage unit (50) in the medical image capturing apparatus (2);
transmitting (504), by the second control unit (22), an examination complete executive instruction to the medical image capturing apparatus (2);
in response to receiving the examination complete executive instruction, changing (205), by the first control unit (13), the examination status of the medical image capturing apparatus (2) to examination complete, so that the examination status of the medical image capturing apparatus (2) stored in the first examination status storage unit (50) and the examination status of the server (5) stored in the second examination status storage unit (60) correspond,

5. The control method according to claim 4, further comprising transmitting (226), by the control unit (13) provided in the medical image capturing apparatus (2), the medical image acquired by the medical image capturing apparatus (2) to the server (5) at specified time intervals while under examination.

6. The control method according to claim 4, further comprising checking a controlled state of the medical image in the server side by sending, by the control unit (13), a request (261) for storage of a medical image to the server (5) that is recognized as unsent in the medical image capturing apparatus (2) to the server (5) at the communication reestablishment after the communication interruption, and selectively transmitting the medical image which is unsent from the medical image capturing apparatus to the server (5).

## Patentansprüche

1. Medizinisches System (1) umfassend einen medizinischen Bilderfassungsapparat (2) und einen Server (5) eingerichtet zum Aufnehmen eines von dem medizinischen Bilderfassungsapparat (2) übermittelten medizinischen Bildes, wobei das medizinische System umfasst:
eine erste Untersuchungsstatus-Speichereinheit (50) des medizinischen Bilderfassungsapparats (2), eingerichtet zum Speichern eines Untersuchungsstatus von in-Untersuchung oder Untersuchung-fertig;
eine zweite Untersuchungsstatus-Speichereinheit (60) des Servers (5), eingerichtet zum Speichern eines Untersuchungsstatus von in-Untersuchung oder Untersuchung-fertig;
eine erste Steuereinheit (13) des medizinischen Bilderfassungsapparats (2), eingerichtet zum Benachrichtigen des Servers (5) über den in der ersten Untersuchungsstatus-Speichereinheit (50) in dem medizinischen Bilderfassungsapparat (2) gespeicherten Untersuchungsstatus;
eine zweite Steuereinheit (22) des Servers (5), eingerichtet zum Erzeugen eines Untersuchung-fertig-Ausführungsbefehls, welcher den medizinischen Bilderfassungsapparat (2) dazu veranlasst, den Untersuchungsstatus des medizinischen Bilderfassungsapparats (2) zu ändern, sodass der in der ersten Untersuchungsstatus-Speichereinheit (50) gespeicherte Untersuchungsstatus des medizinischen Bilderfassungsapparats (2) und der in der zweiten Untersuchungsstatus-Speichereinheit (60) gespeicherte Untersuchungsstatus des Servers (5) übereinstimmen,
wobei, wenn eine Kommunikationswiederherstellung nachdem die Kommunikation mit dem Server (5) unterbrochen war, von der ersten Steuereinheit (13) detektiert wird, und wenn ein in-Untersuchung-Status in der ersten Untersuchungsstatus-Speichereinheit (50) gespeichert ist und ein Untersuchung-fertig-Status in der zweiten Untersuchungsstatus-Speichereinheit (60) gespeichert ist:
die zweite Steuereinheit (22) ist dazu eingerichtet, den medizinischen Bilderfassungsapparat (2) zu instruieren, die derzeitig laufende Untersuchung zu beenden, und
in Erwiderung auf die Instruktion von der zweiten Steuereinheit (22), ist die erste Steuereinheit (13) des medizinischen Bilderfassungsapparats (2) dazu eingerichtet, den in der ersten Untersuchungsstatus-Speichereinheit (50) gespeicherten Untersuchungsstatus des medizinischen Bilderfassungsapparats (2) auf Untersuchung-fertig zu ändern.

2. Medizinisches System gemäß Anspruch 1, bei dem die erste Steuereinheit (13) ferner eingerichtet ist zum Übermitteln des von dem medizinischen Bilderfassungsapparat (2) aufgenommenen medizinischen Bildes an den Server (5) zu spezifizierten Zeitintervallen während der Untersuchung.

3. Medizinisches System gemäß Anspruch 1, bei dem die erste Steuereinheit (13) ferner eingerichtet ist zum Überprüfen eines kontrollierten Zustands des medizinischen Bildes auf der Server-Seite durch Senden einer Anfrage zum Speichern eines ungesendeten medizinischen Bildes des medizinischen Bilderfassungsapparats (2) zu dem Server (5) wenn die Kommunikation wiederhergestellt ist, nach einer Kommunikationsunterbrechung zwischen dem medizinischen Bilderfassungsapparat (2) und dem Server (5), und selektives Übermitteln des medizinischen Bildes, welches ungesendet ist, zu dem Server (5).

4. Kommunikationssteuerverfahren für ein Medizinisches System (1) umfassend einen medizinischen Bilderfassungsapparat (2), welcher eine erste Steuereinheit (13) aufweist, und einen Server (5), welcher eine zweite Steuereinheit (22) aufweist, wobei der medizinische Bilderfassungsapparat (2) eine erste Untersuchungsstatus-Speichereinheit (50) aufweist zum Speichern eines Untersuchungsstatus von in-Untersuchung oder Untersuchung-fertig, wobei der Server (5) eine zweite Untersuchungsstatus-Speichereinheit (60) aufweist zum Speichern eines Untersuchungsstatus von in-Untersuchung oder Untersuchung-fertig, und wobei der Server (5) ein von dem medizinischen Bilderfassungsapparat (2) übermitteltes medizinisches Bild speichert, wobei das Verfahren umfasst:
wenn eine Kommunikationswiederherstellung nachdem die Kommunikation mit dem Server (5) unterbrochen war, von der ersten Steuereinheit (13) detektiert wird, und wenn ein in-Untersuchung-Status in der ersten Untersuchungsstatus-Speichereinheit (50) gespeichert ist und ein Untersuchung-fertig-Status in der zweiten Untersuchungsstatus-Speichereinheit (60) gespeichert ist:
Benachrichtigen (206), von der ersten Steuereinheit (13), des Servers (5) über den in der ersten Untersuchungsstatus-Speichereinheit (50) in dem medizinischen Bilderfassungsapparat (2) gespeicherten Untersuchungsstatus;
Übermitteln (504), von der zweiten Steuereinheit (22), eines Untersuchung-fertig-Ausführungsbefehls an den medizinischen Bilderfassungsapparat (2);
in Erwiderung auf Empfangen des Untersuchung-fertig-Ausführungsbefehls, Ändern (205), von der ersten Steuereinheit (13), des Untersuchungsstatus des medizinischen Bilderfassungsapparats (2) auf Untersuchung-fertig, sodass der in der ersten Untersuchungsstatus-Speichereinheit (50) gespeicherte Untersuchungsstatus des medizinischen Bilderfassungsapparats (2) und der in der zweiten Untersuchungsstatus-Speichereinheit (60) gespeicherte Untersuchungsstatus des Servers (5) übereinstimmen.

5. Steuerverfahren gemäß Anspruch 4, ferner umfassend Übermitteln (226), von der in dem medizinischen Bilderfassungsapparat (2) vorgesehenen ersten Steuereinheit (13), des von dem medizinischen Bilderfassungsapparat (2) aufgenommenen medizinischen Bildes an den Server (5) zu spezifizierten Zeitintervallen während der Untersuchung.

6. Steuerverfahren gemäß Anspruch 4, ferner umfassend Überprüfen eines kontrollierten Zustands des medizinischen Bildes auf der Server-Seite durch Senden, von der Steuereinheit (13), einer Anfrage (261) zum Speichern eines medizinischen Bildes an den Server (5), welches als ungesendet in dem medizinischen Bilderfassungsapparat (2) an den Server (5) erkannt wird, bei der Kommunikationswiederherstellung nach der Kommunikationsunterbrechung, und selektives Übermitteln des medizinischen Bildes, welches ungesendet ist, von dem medizinischen Bilderfassungsapparat zu dem Server (5).

## Revendications

1. Système médical (1) comprenant un appareil (2) de capture d'image médicale et un serveur (5) configuré pour enregistrer une image médicale transmise depuis l'appareil (2) de capture d'image médicale, le système médical comprenant :
une première unité (50) de stockage de statut d'examen de l'appareil (2) de capture d'image médicale configurée pour stocker un statut d'examen examen en cours ou examen terminé ;
une deuxième unité (60) de stockage de statut d'examen du serveur (5) configurée pour stocker un statut d'examen examen en cours ou examen terminé ;
une première unité (13) de commande de l'appareil (2) de capture d'image médicale configurée pour notifier le serveur (5) du statut d'examen stocké dans la première unité (50) de stockage de statut d'examen dans l'appareil (2) de capture d'image médicale ;
une deuxième unité (22) de commande du serveur (5) configurée pour générer une instruction exécutive d'examen terminé qui déclenche l'appareil (2) de capture d'image médicale pour changer le statut d'examen de l'appareil (2) de capture d'image médicale de façon à ce que le statut d'examen de l'appareil (2) de capture d'image médicale stocké dans la première unité (50) de stockage de statut d'examen et le statut d'examen du serveur (5) stocké dans la deuxième unité (60) de stockage de statut d'examen correspondent,
dans lequel, lorsqu'un rétablissement de communication après qu'une communication avec le serveur (5) a été coupée est détecté par la première unité (13) de commande, et lorsqu'un statut d'examen en cours est stocké dans la première unité (50) de stockage de statut d'examen et qu'un statut d'examen terminé est stocké dans la deuxième unité (60) de stockage de statut d'examen :
la deuxième unité (22) de commande est configurée pour indiquer à l'appareil (2) de capture d'image médicale de mettre fin à l'examen alors en cours, et
en réponse à l'instruction de la deuxième unité (22) de commande, la première unité (13) de commande de l'appareil (2) de capture d'image médicale est configurée pour passer l'état de l'appareil (2) de capture d'image médicale stocké dans la première unité (50) de stockage de statut d'examen à examen terminé.

2. Système médical selon la revendication 1, dans lequel la première unité (13) de commande est en outre configurée pour transmettre l'image médicale acquise par l'appareil (2) de capture d'image médicale au serveur (5) à intervalles de temps spécifiés tant que l'examen est en cours.

3. Système médical selon la revendication 1, dans lequel la première unité (13) de commande est en outre configurée pour contrôler un état commandé de l'image médicale dans le côté serveur en envoyant une requête de stockage d'une image médicale non envoyée de l'appareil (2) de capture d'image médicale au serveur (5) lorsqu'une communication est rétablie après une interruption de communication entre l'appareil (2) de capture d'image médicale et le serveur (5), et en transmettant sélectivement l'image médicale qui est non envoyée au serveur (5).

4. Procédé de commande de communication pour un système médical (1) comprenant un appareil (2) de capture d'image médicale ayant une première unité (13) de commande et un serveur (5) ayant une deuxième unité (22) de commande, dans lequel l'appareil (2) de capture d'image médicale a une première unité (50) de stockage de statut d'examen pour stocker un statut d'examen examen en cours ou examen terminé, dans lequel le serveur (5) a une deuxième unité (60) de stockage de statut d'examen pour stocker un statut d'examen examen en cours ou examen terminé, et dans lequel le serveur (5) enregistre une image médicale transmise depuis l'appareil (2) de capture d'image médicale, le procédé comprenant :
lorsqu'un rétablissement de communication après qu'une communication avec le serveur (5) a été coupée est détecté par la première unité (13) de commande, et lorsqu'un statut d'examen en cours est stocké dans la première unité (50) de stockage de statut d'examen et qu'un statut d'examen terminé est stocké dans la deuxième unité (60) de stockage de statut d'examen :
la notification (206), par la première unité (13) de commande, au serveur (5) du statut d'examen stocké dans la première unité (50) de stockage de statut d'examen dans l'appareil (2) de capture d'image médicale ;
la transmission (504), par la deuxième unité (22) de commande, d'une instruction exécutive d'examen terminé à l'appareil (2) de capture d'image médicale ;
en réponse à la réception de l'instruction exécutive d'examen terminé, le passage (205), par la première unité (13) de commande, du statut d'examen de l'appareil (2) de capture d'image médicale à examen terminé, de façon à ce que le statut d'examen de l'appareil (2) de capture d'image médicale stocké dans la première unité (50) de stockage de statut d'examen et le statut d'examen du serveur (5) stocké dans la deuxième unité (60) de stockage de statut d'examen correspondent.

5. Procédé de commande selon la revendication 4, comprenant en outre la transmission (226), par l'unité (13) de commande prévue dans l'appareil (2) de capture d'image médicale, de l'image médicale acquise par l'appareil (2) de capture d'image médicale au serveur (5) à intervalles de temps spécifiés tant que l'examen est en cours.

6. Procédé de commande selon la revendication 4, comprenant en outre le contrôle d'un état commandé de l'image médicale dans le côté serveur en envoyant, par l'unité (13) de commande, une requête (261) de stockage d'une image médicale au serveur (5) qui est reconnue comme non envoyée dans l'appareil (2) de capture d'image médicale au serveur (5) au rétablissement de communication après l'interruption de communication, et en transmettant sélectivement l'image médicale qui est non envoyée de l'appareil de capture d'image médicale au serveur (5).
